# EUROPÄISCHE PATENTSCHRIFT

(11) **EP 1 740 096 B1**
(45) Veröffentlichungstag und Bekanntmachung des Hinweises auf die Patenterteilung: **17.10.2012**
(21) Anmeldenummer: 05748395.0
(22) Anmeldetag: 21.04.2005
(51) Int. Cl.: A61B 5/15

(54) **BLUTENTNAHMEVORRICHTUNG, INSBESONDERE FÜR NEUGEBORENE UND KLEINKINDER ODER KLEINTIERE**
BLOOD TAKING DEVICE IN PARTICULAR FOR NEW-BORN BABIES AND SMALL CHILDREN OR SMALL ANIMALS
DISPOSITIF DE PRELEVEMENT DE SANG, EN PARTICULIER POUR NOUVEAU-NES ET PETITS ENFANTS OU PETITS ANIMAUX

(30) Priorität: 30.04.2004 DE 102004021741
(43) Veröffentlichungstag der Anmeldung: 10.01.2007
(73) Patentinhaber: Sarstedt AG & Co., 51588 Nümbrecht (DE)
(72) Erfinder: SARSTEDT, Walter, 51588 Nümbrecht (DE)
(74) Vertreter: Grosse, Wolf-Dietrich Rüdiger
(86) Internationale Anmeldenummer: PCT/DE2005/000737
(87) Internationale Veröffentlichungsnummer: WO 2005/104947

(56) Entgegenhaltungen:
- US-A- 4 595 021
- US-A- 4 653 511
- US-A- 4 703 763

## Beschreibung

Die Erfindung betrifft eine Blutentnahmevorrichtung für Neugeborene und Kleinkinder oder Kleintiere, umfassend ein Blutentnahmegefäß, das an seinem vorderen Ende mit einer eine angeschärfte Spitze aufweisenden Kanüle versehen ist, einen in das Blutentnahmegefäß eintauchenden Stößel und ein in das Blutentnahmegefäß in definierter Position eingedrücktes, luftdurchlässiges, den Fluss des entnommenen Blutes automatisch stoppendes, poröses Begrenzungselement aufweist, wobei das Begrenzungselement mit unverändert eingehaltener Positionierung angeordnet ist, wie durch die US 4.595.021 A bekanntgeworden

Eine damit vergleichbare, zur Blutentnahme ebenfalls eine Injektionsnadel aufweisende Blutentnahmevorrichtung ist aus der US 4.703.763 A bekannt und dient zur Gewinnung von arteriellen Blutproben zur Blutgasanalytik, d.h. von Blutentnahmen bei einem sehr hohen Druck. Das poröse Begrenzungselement stellt im abgedichteten Zustand einen Kolben dar, der mittels des Stößels mechanisch nach vorne herausbewegt wird, um die Blutprobe am distalen Ende abzugeben.

Bei einer aus der US 4.623.511 A bekannten Blutentnahmevorrichtung wird über ein kapillares Leitungsstück eine Mischkammer mit einer davon weit entfernten Filteranordnung verbunden.

Durch die DE 39 32 112 C2 ist eine Blutentnahmevorrichtung mit einem an seinem hinteren Ende hermetisch und an seinem vorderen Ende bis auf eine Entlüftung geschlossenen äußeren Blutentnahmegefäß bekanntgeworden, das konzentrisch ein inneres Probenröhrchen aufnimmt. Das Probenröhrchen ist an seinem vorderen Ende mit einer in die Vene eines Patienten einstechbaren Kanüle versehen, deren hinteres Ende in Strömungsverbindung mit dem Blutentnahmegefäß steht. Die Kanüle ist in einem Halter bzw. Lueransatz angeordnet, der auf einen Konus des Probenröhrchens aufgesteckt wird. Diese aus äußerem Blutentnahmegefäß und innerem Probenröhrchen bestehende Blutentnahmevorrichtung ist sehr großbauend, daher trotz transparentem Material für das Blutentnahmegefäß und das Probenröhrchen unübersichtlich, was die Beobachtung des Blutflusses erschwert, und in seiner Handhabung umständlich. Außerdem ist es schwierig, von der entnommenen großen Blutmenge definierte, kleine Probenmengen zu Untersuchungszwecken zu entnehmen.

Nach einer Venenblutentnahme muß zunächst der die Kanüle tragende, aufgesteckte Halter bzw. die Luerkanüle sorgfältig abgenommen und entsorgt werden. Dies birgt eine Verletzungsgefahr an der zudem unvermeidlich mit Blut benetzten Kanüle in sich. Anschließend wird das innere, kapillarartige Probenröhrchen herausgezogen, um die entnommene Blutmenge in das äußere Blutentnahmegefäß zu entleeren. Beim Herausziehen des Probenröhrchens lässt sich ebenfalls nicht vermeiden, dass Blut mit abgezogen wird.

Abgesehen davon, ist diese bekannte Blutentnahmevorrichtung bei Entnahme von nur geringen Blutmengen und insbesondere bei Neugeborenen und Kleinkindern bzw. Kleintieren aufgrund deren anderer physiologischen Bedingungen, insbesondere des sehr viel geringeren Venendruckes, nicht geeignet. Denn der geringe Blutdruck dieser Patienten reicht nicht aus, um das Blut, das zunächst durch den Hohlraum der Kanüle läuft, gegen die in dem sich anschließenden Probenröhrchen unvermeidliche Luft in Fluss zu halten, so dass der Blutfluss in der Kanüle zum Stocken kommt. Zur Blutentnahme bei Neugeborenen oder dergleichen wird deshalb nur die zuvor abgezogene Luerkanüle verwendet, deren Hohlraum des Aufsteckansatzes die entnommene Blutmenge aufnimmt. Die Blutentnahme mit nur dem Lueransatz ist sehr schwierig und setzt beim Anwender viel Erfahrung voraus. Schließlich ist die in dem Lueransatz bezogen auf das geringe Probenvolumen bei der Entleerung in ein Probengefäß verbleibende Restmenge Blut zu groß, und eine definierte Entnahme einer Probenmenge ist schon gar nicht möglich.

Das Vorgenannte gilt gleichermaßen dann, wenn insbesondere zur Vermeidung der im Lueransatz verbleibenden Restmenge der Lueransatz entfernt und dann nur die Kanüle zum Einsatz kommt. Aus dem offenen, eventuell auch gebogenen rückwärtigen Ende der Kanüle kann die Blutprobe dann direkt in ein Probengefäß abtropfen. Da es sehr problematisch ist, ein Auffanggefäß in einer geeigneten Weise unter das offene Kanülenende zu halten, um die Blutprobe sicher auffangen zu können, hält eine Person den Patienten und führt die Punktion durch, während eine andere Person das Probengefäß zum Aufnehmen des Blutes hält.

Zur Verbesserung der Entnahme von venösem Blut bei Neu- bzw. Frühgeborenen, Säuglingen und Kleinkindern ist es durch die DE 100 603 02 A1 bekanntgeworden, eine an ihrem distalen Ende mit einer angeschärften Spitze ausgebildete Hohlnadel bzw. Kanüle an ihrem hinteren, proximalen Ende mit einem seitlich zu ihrer Längsachse abgewinkelten Blutauslaß zu versehen. Die Kanüle ist in einem Halter angeordnet, dessen hinter dem Blutauslaß liegender Griffbereich eine Anfaß- bzw. Führungsmöglichkeit bietet. Bei der Blutentnahme wird unter die Auslaßöffnung der Kanüle ein das ausfließende Blut auffangendes Gefäß gehalten. Anders als bei herkömmlich eingesetzten Injektionsnadeln braucht die Kanüle dieser Blutentnahmevorrichtung nicht mehr besonders präpariert zu werden, z.B. ist kein Abbrechen eines Luer-Konus mehr erforderlich. Allerdings ist eine visuelle Kontrolle der zu entnehmenden Blutmenge nur sehr unzureichend möglich, weil bei diesen Blutentnahmevorrichtungen von nur geringer Größe das Griffstück und/oder die Finger einer das Blut entnehmenden Person die Sicht auf die Auslassöffnung erheblich einschränken, möglicherweise sogar völlig verdecken. Die seitlich abgewinkelte Auslassöffnung führt außerdem dazu, dass die Kanüle nur in einer .Gebrauchslage einen optimalen Blutauslaß ermöglicht. Abgesehen davon, dass die Herstellung einer gekrümmten, seitlich abgewinkelten Kanüle aufwendig ist, liegt zudem auch der Nachteil vor, dass der durch die Krümmung bedingte höhere Strömungswiderstand der Kanüle den Blutfluß des ohnehin nur in einer geringen Menge entnommenen Blutes behindert.

Der Erfindung liegt daher die Aufgabe zugrunde, eine gattungsgemäße Blutentnahmevorrichtung ohne die genannten Nachteile zu schaffen, dabei insbesondere die Anwender- und Bedienungsfreundlichkeit zu verbessern, und die sichere Entnahme auch geringer Blutmengen zu ermöglichen.

Diese Aufgabe wird erfindungsgemäß dadurch gelöst, daß das vordere, die Kanüle und das Begrenzungselement aufweisende Ende des Blutentnahmegefäßes als eine auch bei einem niedrigen Venendruck eine Blutentnahme ermöglichende Kapillare ausgebildet ist, wobei das poröse Begrenzungselement von einem Anschlag des Blutentnahmegefäßes in definierter, unverändert eingehaltener Positionierung festgelegt ist, und das hintere Ende des Blutentnahmegefäßes von einem abgedichtet in dem Blutentnahmegefäß geführten, zur Blutentnahme temporär lüftbaren Stößel verschlossen ist. Es lässt sich damit eine sehr schmalbauende, keine besondere Hantierung an der Kanüle erfordernde Vorrichtung erreichen, bei der sich der Blutfluß unbeeinträchtigt beobachten lässt und die keine Blutverluste mit sich bringt sowie eine genau definierte Blutentnahme und Abgabe einer exakt gewünschten Probenmenge ermöglicht. Die Kanüle kann einstückig mit dem Blutentnahmegefäß ausgebildet sein oder aufsteckbar damit verbunden werden.

Das luftdurchlässige Begrenzungselement stoppt eine weitere Blutaufnahme schon beim ersten Blutkontakt, d.h. sobald das festgelegte Füllvolumen erreicht ist. Das Begrenzungselement sorgt für eine exakte Volumenbestimmung der aufzunehmenden Blutprobe, da es eine definierte und unverändert eingehaltene Positionierung, vorzugsweise durch einen inneren Anschlag, gegen den das Begrenzungselement bei der Herstellung gezielt eingeführt und eingedrückt wird, in dem Blutentnahmegefäß erfährt. Hiervon völlig abweichend sind Sinn und Zweck bei z.B. in Pipettenspitzen vorgesehenen, an sich bekannten porösen Filterelementen. Das Filterelement soll dort eine Kontamination der Pipette, durch z.B. Aerosole, verhindern, wobei ein Kontakt mit der aufgenommenen Flüssigkeit nicht erwünscht wird. Auch ist die exakte Einhaltung einer definierten Filterposition für die Gewährleistung der Funktionalität einer solchen Pipettenspitze unmaßgeblich.

Die erfindungsgemäße Blutentnahmevorrichtung erlaubt zudem, die in dem Blutentnahmegefäß einschließlich dem Hohlraum der Kanüle bestimmt aufgenommene Blutmenge zur Analyse in der gewünschten Dosierung herauszudrücken, da die Blutmenge durch einen Stößelhub völlig, alternativ aber auch gestuft dosiert, entleert werden kann. Die Belüftbarkeit des Stößels, z.B. über eine vom Lieferzustand der Blutentnahmevorrichtung her und somit in der Blutentnahme- bzw. Blutaufnahmeposition undichten Stößelhalterung oder durch eine Entlüftungsbohrung im Stößel, vorzugsweise an dessen oberen Ende, schafft die für die Entnahme der Probenmenge notwendige Entlüftung. Hingegen kommt die erforderliche Abdichtung zwischen Stößel und Gefäßgehäuse zur Zwangsentleerung durch Überdruck mit Aufhebung der vorgesehenen Entlüftung zustande, und zwar beim Verschieben des Stößels aus der entlüfteten, undichten Ausgangsposition in die abgedichtete Abgabestrecke oder durch Verschließen der Bohrung mittels Finger bei von vornherein abgedichtet angeordnetem Stößel.

Ein Vorschlag der Erfindung sieht vor, dass das vordere, die Kanüle und das Begrenzungselement aufweisende Ende des Blutentnahmegefäßes als Kapillare ausgebildet ist. Die Kapillare unterstützt das Einsaugen des Blutes in den von dem Filterelement begrenzten Aufnahmeraum der Blutentnahmevorrichtung.

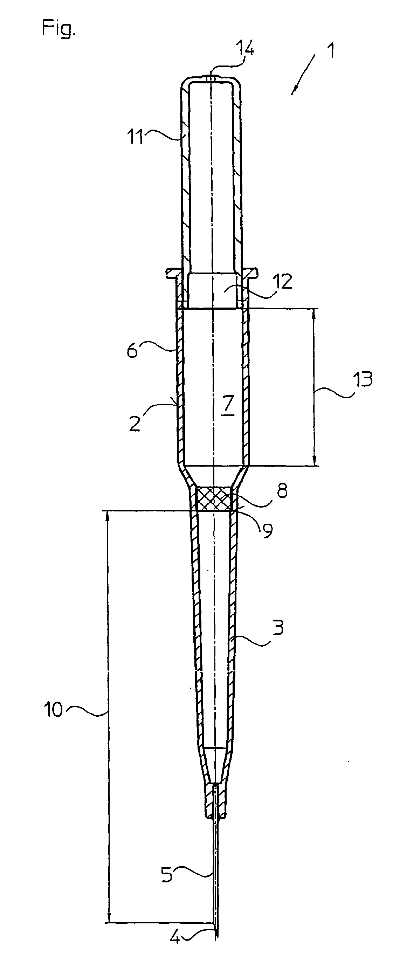

Weitere Merkmale und Einzelheiten der Erfindung ergeben sich aus den Ansprüchen und der nachfolgenden Beschreibung eines in der einzigen Zeichnung als Gesamtansicht in einem Längsschnitt dargestellten Ausführungsbeispiels der Erfindung.

Eine in der Zeichnungsfigur dargestellte Blutentnahmevorrichtung 1 besitzt ein Blutentnahmegefäß 2, das im Ausführungsbeispiel an seinem vorderen Ende in trompetenartiger Form eine Kapillare 3 mit einer darin angeordneten, an ihrer Spitze 4 angeschärften Kanüle 5 aufweist und einstückig in einen sich nach hinten anschließenden, im Durchmesser sehr viel größeren Zylinderabschnitt 6 übergeht. Die Kapillare 3 wird vom Kammervolumen 7 des Zylinderabschnitts 6 durch ein poröses Begrenzungselement 8 getrennt, das definiert gegen einen Anschlag 9 des Blutentnahmegefäßes 2 eingedrückt ist und das Aufnahmevolumen der Kapillare 3 aufgrund dieser genauen Positionierung exakt bestimmt. Sobald das entnommene Blut das Begrenzungselement 8 erreicht hat, wird das Aufsaugen über die Kanüle 5 gestoppt. Es kann somit ein Probenvolumen 10 ganz genau ermittelt bzw. festgelegt werden. Durch die direkte Anbringung der Kanüle 5 an dem vorderen Ende der Blutentnahmevorrichtung 1, hier der Kapillare 3 des Blutentnahmegefäßes 2, werden jegliche den Blutfluß und die Handhabung störende Konturübergänge vermieden. Da die Blutentnahmevorrichtung 1 zudem in sich geschlossen ist, wird eine Kontaminationsgefahr vermieden.

In dem von Kapillare 3 entfernten Ende des Blutentnahmegefäßes 2 ist ein in seiner Ausgangsposition zur Blutaufnahme bzw. -entnahme gezeigter Stößel 11 angeordnet. Zur Entlüftung bei der Blutaufnahme ist ein in den Zylinderabschnitt 6 eintauchender Stößelkopf 12 von vornherein abgedichtet in dem Blutentnahmegefäß 2 angeordnet. Diesen abgedichteten Eingriff hält er dann auch während des Hubes über eine Abgabestrecke 13 zum Entleeren der aufgenommenen Blutmenge ein, wozu eine zur Blutaufnahme im gezeigten Ausgangszustand des Stößels 11 an dessen oberen Ende vorgesehene Entlüftungsbohrung 14 während des Verschiebens mittels Auflegen eine Fingers von einer Bedienungsperson verschlossen wird. Alternativ könnte der Stößelkopf 12 mit einem Luftspalt zur Gefäßinnenwand ausgebildet werden, der sich durch eine beispielsweise komplementäre konstruktive Gestaltung der einander zugewandten Wände von Stößelkopf 9 und Innenwand des Zylinderabschnitts 6 mit dem Eintreten des Stößelkopfes 12 in die Abgabestrecke 13 schließt.

Zur Entnahme einer Probenmenge lässt sich die durch den Stopp des Begrenzungselementes 8 in der Kapillare 3 einschließlich Hohlraum der Kanüle 5 definiert aufgenommene Blutmenge in einfacher Weise durch den Stößel 12 als vorzugsweise dosierte Gesamtmenge, aber auch als dosierte Teilmenge, in ein separates Probengefäß (nicht gezeigt) herausdrücken, in das die Probenmenge über die Kanüle 5 einfließt. Hierbei ist eine rückstandsfreie Entleerung ohne Verlust an Probenmenge möglich, was bei der Entnahme nur geringer Blutmengen besonders wichtig ist.

## Patentansprüche

1. Blutentnahmevorrichtung (1) für Neugeborene und Kleinkinder oder Kleintiere, umfassend ein Blutentnahmegefäß (2), das an seinem vorderen Ende mit einer eine angeschärfte Spitze (4) aufweisenden Kanüle (5) versehen ist, einen in das Blutentnahmegefäß (2) eintauchenden Stößel (11) und ein in das Blutentnahmegefäß (2) in definierter Position eingedrücktes, luftdurchlässiges, den Fluss des entnommenen Blutes automatisch stoppendes, poröses Begrenzungselement (8) aufweist, wobei das Begrenzungselement (8) mit unverändert eingehaltener Positionierung angeordnet ist, und wobei der Stössel geeignet ist, zur Abgabe des entnommenen Blutes in ein Probengefäß zumindest eine Teilmenge des vor dem Begreuzungselement eingeschlossenen Blutes durch überdruck zu entleeren,
**dadurch gekennzeichnet,**
**daß** das vordere, die Kanüle (5) und das Begrenzungselement (8) aufweisende Ende des Blutentnahmegefäßes (2) als eine auch bei einem niedrigen Venendruck eine Blutentnahme ermöglichende Kapillare (3) ausgebildet ist, wobei das poröse Begrenzungselement (8) von einem Anschlag (9) des Blutentnahmegefäßes (2) in der definierten, unverändert eingehaltenen Positionierung festgelegt ist, und das hintere Ende des Blutentnahmegefäßes (2) von dem abgedichtet in dem Blutentnahmegefäß (2) geführten, zur Blutentnahme temporär lüftbaren Stößel (11) verschlossen ist.

## Claims

1. A blood collection device (1) for new-born babies and small children or small animals, comprising a blood collection vessel (2), which is provided at its front end with a cannula (5) having a scarfed tip (4), a plunger (11) dipping into the blood collection vessel (2), and an air-permeable, porous delimiting element (8), which is pushed into the blood collection vessel (2) in a defined position and automatically stops the flow of removed blood, said delimiting element (8) being arranged with unchanged, maintained positioning and the plunger being adapted to empty, by overpressure, at least some of the blood trapped by the delimiting element to deliver the removed blood into a sample vessel,
**characterised in that**
the front end of the blood collection vessel (2) having the cannula (5) and the delimiting element (8) is formed as a capillary (3) making it possible to collect blood, even at low venous pressure, the porous delimiting element (8) being fixed by a stop (9) of the blood collection vessel (2) in the defined, unchanged, maintained positioning and the rear end of the blood collection vessel (2) being closed by the plunger 11), which is guided in the blood collection vessel (2) in a sealed manner and which can be vented temporarily for blood collection.

## Revendications

1. Dispositif de prise de sang (1) pour nouveau-nés et enfants en bas âge ou jeunes animaux, comprenant un réceptacle de prise de sang (2) qui est pourvu à son extrémité antérieure d'une canule (5) présentant une pointe acérée (4), d'un piston (11) plongeant dans le réceptacle de prise de sang (2) et d'un élément limiteur poreux (8) enfoncé dans le réceptacle de prise de sang (2) en position définie, laissant passer l'air et arrêtant automatiquement le flux de sang prélevé, l'élément limiteur (8) étant disposé avec un positionnement maintenu sans changement et le piston étant apte, pour délivrer le sang prélevé dans un réceptacle d'échantillon, à vider par surpression au moins une quantité partielle du sang circonscrit par l'élément limiteur,
**caractérisé en ce que**
l'extrémité antérieure présentant la canule (5) et l'élément limiteur (8) du réceptacle de prise de sang (2) est réalisée sous forme d'un capillaire (3) permettant une prise de sang même en cas de pression veineuse faible, l'élément limiteur poreux (8) étant bloqué par une butée (9) du réceptacle de prise de sang (2) dans le positionnement défini maintenu sans changement et l'extrémité postérieure du réceptacle de prise de sang (2) étant fermée par le piston (11) guidé de manière étanche dans le réceptacle de prise de sang (2) et pouvant être ventilé temporairement pour la prise de sang.
